# EUROPÄISCHE PATENTSCHRIFT

(11) **EP 2 271 262 B1**
(45) Veröffentlichungstag und Bekanntmachung des Hinweises auf die Patenterteilung: **23.01.2019**
(21) Anmeldenummer: 09741827.1
(22) Anmeldetag: 28.04.2009
(51) Int. Cl.: A61B 5/155

(54) **LANZETTENRAD UND VERFAHREN ZUR HERSTELLUNG EINES LANZETTENRADES**
LANCET WHEEL AND METHOD FOR MANUFACTURING A LANCET WHEEL
ROUE DE LANCETTE ET PROCÉDÉ DE FABRICATION D'UNE ROUE DE LANCETTE

(30) Priorität: 03.05.2008 EP 08008393
(43) Veröffentlichungstag der Anmeldung: 12.01.2011
(73) Patentinhaber: Roche Diabetes Care GmbH, 68305 Mannheim (DE); F. Hoffmann-La Roche AG, 4070 Basel (CH)
(72) Erfinder: KEIL, Michael, 67071 Ludwigshafen (DE)
(74) Vertreter: Mommer, Niels
(86) Internationale Anmeldenummer: PCT/EP2009/003057
(87) Internationale Veröffentlichungsnummer: WO 2009/135608

(56) Entgegenhaltungen:
- EP-A- 0 985 376
- EP-A- 1 880 671
- EP-A- 2 050 392
- WO-A-2005/118690
- WO-A-2007/060004

## Beschreibung

Die Erfindung geht aus von einem Lanzettenrad mit den im Oberbegriff des Anspruchs 1 angegebenen Merkmalen, wie es aus der EP 1 360 934 A1 bekannt ist. Derartige Lanzettenräder werden beispielsweise für Stechsysteme benötigt, mit denen Diabetiker Körperflüssigkeitsproben zur Bestimmung des Glukosegehalts gewinnen.

Die Lanzetten des aus der EP 1 360 934 A1 bekannten Lanzettenrades sind mit einem Träger über beidseitig an dem Lanzettenkörper befestigte Stege verbunden und in Umfangsrichtung orientiert. Zum Aufrichten der Lanzetten in ihre Gebrauchsstellung werden die Stege tordiert. Vor dem Aufrichten liegen die Lanzetten in der Ebene des Lanzettenrades, so dass die Lanzettenspitzen geschützt sind und sich das Lanzettenrad insbesondere beim Einlegen in ein Stechgerät gut Handhaben lässt.

Dies ist ein wichtiger Vorteil gegenüber aus der WO 2005/118690 bekannten Lanzettenrädern, bei denen Lanzetten zum Gebrauch mit einem Kniehebel in der Ebene des Lanzettenrades bewegt werden, so dass die Gefahr besteht, dass man sich beim Handhaben eines solchen Lanzettenrades an einer Lanzettenspitze verletzt.

Das aus der EP 1 369 934 A1 bekannte Lanzettenrad enthält Lanzetten, in die ein elektrochemischer Sensor zum Messung einer Analytkonzentration integriert ist. Die Lanzetten haben einen Lanzettenkörper aus einem Substrat, auf welches zur Ausbildung einer Elektrodenfläche ein Metallfilm aufgebracht ist. Der Lanzettenkörper trägt eine Reaktionszone mit Nachweischemikalien, die von einem weiteren Substratplättchen bedeckt ist, das zur Ausbildung der zweiten Elektrodenfläche ebenfalls metallisiert ist. Diese Lanzetten sind in Kunststoffkörper eingebettet, die jeweils über zwei Metallstege mit Kunststoffringen des Lanzettenrades verbunden sind.

Nachteilig an einem Stechsystem mit den aus der EP 1 360 934 A1 bekannten Lanzettenrädern sind jedoch relativ hohe Kosten.

Aufgabe der Erfindung ist es, einen Weg aufzuzeigen, wie ein Stechsystem mit Lanzettenrädern gemäß dem Oberbegriff des Anspruchs 1 kostengünstiger verwirklicht werden kann.

Diese Aufgabe wird erfindungsgemäß durch ein Lanzettenrad mit den im Anspruch 1 angegebenen Merkmalen gelöst. Vorteilhafte Weiterbildungen der Erfindung sind Gegenstand der Unteransprüche. Die Aufgabe wird auch durch ein Verfahren zur Herstellung eines solchen Lanzettenrads mit den im Anspruch 10 angegebenen Merkmalen gelöst.

Wegen der radialen Ausrichtung der Lanzetten mit radial verlaufenden Stegen kann ein erfindungsgemäßes Lanzettenrad eine vorteilhaft große Anzahl von Lanzetten aufweisen, was dazu beiträgt, die Kosten des Stechsystems zu senken, da die Kosten pro Lanzette reduziert werden können. Zudem wird für jede Lanzette nur ein einziger Steg benötigt, was wegen einer einfacheren Geometrie die Herstellung des Lanzettenrads vereinfacht und ebenfalls Kosteneinsparungen ermöglicht.

Obwohl ein erfindungsgemäßes Lanzettenrad wesentlich mehr Lanzetten aufweisen kann, als es bei einem aus der EP 1 360 934 A1 bekannten Lanzettenrad möglich ist, können die Vorteile einer ebenen Anordnung in einer Ausgangsstellung, aus der die Lanzetten für einen Stich in eine Gebrauchsstellung aufgerichtet werden, beibehalten werden, indem die Stege, über welche die Lanzetten mit einem Träger des Lanzettenrads verbunden sind, zum Aufrichten der Lanzetten nicht tordiert, sondern gebogen, bevorzugt geknickt, werden und hierzu eine Soll-Biegestelle aufweisen.

Die Soll-Biegestelle kann besonders vorteilhaft ausgebildet werden, indem die Stege eines erfindungsgemäßen Lanzettenrads mit einem Kunststoffkörper versehen werden, der ein Gelenk aufweist und auf diese Weise die Soll-Biegestelle vorgibt. Beispielsweise kann der Kunststoffkörper als Soll-Biegestelle eine Schwächung, beispielsweise in Form einer Kerbe oder Perforation, aufweisen, um welche die Stege zum Aufrichten der Lanzetten gebogen werden.

Bevorzugt sind die Stege eines erfindungsgemäßen Lanzettenrades federnd um die Soll-Biegestelle biegsam, so dass eine aufgerichtete Lanzette aus der Gebrauchsposition nach einem Stich ohne äußere Krafteinwirkung wieder in eine ebene Lage übergeht. Bei dieser ebenen Lage handelt es sich bevorzugt um die Ausgangsposition der Lanzette. Möglich ist es aber auch, dass eine Lanzette aus der Gebrauchsposition in eine ebene Lage schnappt, in der sie umgekehrt wie in der Ausgangsposition orientiert ist. Wegen der federnden Beweglichkeit der Stege kann eine Lanzette in einem Stechgerät mit einer Schwenkeinrichtung, beispielsweise einem die Lanzette aus der Radebene drückenden Stift, in die Gebrauchposition gebracht werden und kehrt von selbst in eine ebene Position zurück, sobald die Schwenkeinrichtung nicht mehr auf sie einwirkt. Der erfindungsgemäße Kunststoffkörper der Stege ermöglicht vorteilhaft elastische Eigenschaften, die zum Aufrichten der Lanzetten eine Biegung um einen rechten Winkel ermöglichen und nach einem Stich ohne äußere Krafteinwirkung für eine Bewegung der Lanzetten in eine ebene Lage sorgen.

Ein erfindungsgemäßes Lanzettenrad kann kostengünstig hergestellt werden, indem ein Lanzettenradrohling, der ringförmig angeordnete Lanzetten mit einem in einer Spitze endenden Lanzettenkörper aufweist, aus Blech ausgeschnitten und anschließend mit Kunststoff umspritzt wird. Das Ausschneiden des Lanzettenradrohlings kann beispielsweise durch Laserschneiden oder Stanzen, besonders vorteilhaft durch Ätzen, erfolgen.

Durch das Umspritzen mit Kunststoff können kostengünstig Kunststoffkörper der die Lanzetten mit einem Träger verbindenden Stege ausgebildet und Soll-Biegestellen zum Aufrichten der Lanzetten vorgegeben werden. Dabei kann auch ein Kunststoffkörper eines mit den Stegen verbundenen Trägers ausgebildet werden. Der Kunststoffkörper des Trägers kann in die Kunststoffkörper der Lanzetten übergehen, so dass die Kunststoffkörper der Stege und der Kunststoffkörper des Trägers integral oder einstückig ausgebildet sind.

Ein erfindungsgemäßes Herstellungsverfahren kommt mit vorteilhaft wenigen Handhabungs- und Arbeitsschritten aus. Ein einstückiger Lanzettenradrohling lässt sich leicht aus Blech ausschneiden und anschließend in ein Spritzgusswerkzeug einlegen. Für ein erfindungsgemäßes Lanzettenrad genügen an sich bereits zwei Teile, nämlich der Lanzettenradrohling und ein um den Lanzettenradrohling gespritzter Kunststoffkörper, der Stege mit einer vorgegebenen Soll-Biegestelle aufweisen kann. Zusätzlich können die Lanzettenspitzen mit vorteilhaft geringem Aufwand mit einem Sterilschutz versehen werden, beispielsweise indem die Lanzettenspitzen mit Schaumstoff umspritzt werden.

Die Metallstege des Lanzettenradrohlings können nach dem Einlegen in ein Spritzgusswerkzeug durchtrennt und/oder entfernt werden, so dass beim Aufrichten der Lanzetten kein Metallteil gebogen werden muss. Der mit dem Durchtrennen der Metallstege verbundene Arbeitsschritt lässt sich aber auch einzusparen, indem die von den Lanzettenkörpern ausgehenden Metallstege in den Kunststoffkörpern der Stege eingebettet werden und sich durch die Soll-Biegestelle hindurch erstrecken. Bevorzugt ist der Kunststoffkörper an der Soll-Biegestelle breiter als der Metallsteg, besonders bevorzugt mindestens doppelt so breit, insbesondere mindestens dreimal so breit. Auf diese Weise wird der zum Aufrichten einer Lanzette erforderliche Kraftaufwand durch den Metallsteg nur unwesentlich erhöht.

Erfindungsgemäß ist vorgesehen, dass der Träger einen Ring aufweist, der die Lanzetten umschließt. Auf diese Weise lassen sich die Lanzetten vor unbeabsichtigtem Kontakt bei der Handhabung des Lanzettenrades schützen. Zudem kann ein Lanzettenrad an einem solchen Ring gut gegriffen werden. Der Ring kann beim Umspritzen des Lanzettenradrohlings mit Kunststoff ohne zusätzlichen Aufwand ausgebildet werden.

Bevorzugt bildet der Träger auf einer Seite des Lanzettenrades einen Anschlag, der ein Aufrichten der Lanzetten nur in einer Richtung zulässt. Der Anschlag kann beispielsweise als eine Ringscheibe ausgebildet sein. Ein solcher Anschlag reduziert als Knickschutz die Gefahr eine Beschädigung des Lanzettenrades oder einer Verletzung bei der Handhabung durch ein versehentliches Biegen eines Steges, da die Lanzetten wegen des Anschlags nur in einer Richtung, nämlich weg von dem Anschlag, aufgerichtet werden können. In ihrer Ausgangsstellung vor dem Aufrichten in die Gebrauchsstellung können die Lanzetten auf dem Anschlag aufliegen oder in einem Abstand davon angeordnet sein.

Eine weitere vorteilhafte Weiterbildung der Erfindung sieht vor, dass das Lanzettenrad in seinem Zentrum ein Loch aufweist, mit dem es beispielsweise auf eine Welle eines Stechgeräts aufgesteckt werden kann.

Eine weitere vorteilhafte Weiterbildung der Erfindung sieht vor, dass die Lanzettenspitzen von einem Sterilschutz umgeben sind. Der Sterilschutz kann beispielsweise um die Lanzettenspitzen herumgespritzt werden, beispielsweise als Schaumstoff. Ein derartiger Sterilschutz wird bei einem Lanzettenstich von selbst von der Lanzettenspitze abgestreift. Ein Sterilschutz lässt sich auch durch Folie ausbilden, beispielsweise indem der Lanzettenradrohling zumindest im Bereich der Lanzettenspitzen beidseitig mit Folie bedeckt wird. Durch anschließendes Umspritzen des Lanzettenradrohlings mit Kunststoff lässt sich die Sterilschutzfolie ohne zusätzlichen Aufwand befestigen. Beim Aufrichten einer Lanzette wird die Sterilschutzfolie im Bereich der betreffenden Lanzette durchbrochen.

Eine weitere vorteilhafte Weiterbildung der Erfindung sieht vor, dass das Lanzettenrad einen Griff aufweist, der sich senkrecht zu einer Radebene, in der die Lanzetten liegen, erstreckt. Ein solcher Griff erleichtert das Einlegen des Lanzettenrades in ein Stechgerät. Der Griff kann über eine Sollbruchstelle mit dem Lanzettenrad verbunden sein. Möglich ist es auch, den Griff separat auszubilden und bei Bedarf mit dem eigentlichen Lanzettenrad zu verbinden, beispielsweise, indem der Griff in ein zentrisches Loch gesteckt wird.

Ein zur Herstellung eines erfindungsgemäßen Lanzettenrades aus Metall ausgeschnittener Lanzettenradrohling mit mehreren ringförmig angeordneten Lanzetten, die radial ausgerichtet sind und einen in einer Lanzettenspitze endenden Lanzettenkörper aufweisen, und einem Träger, mit dem die Lanzetten über radial verlaufende Stege verbunden sind, die ein Aufrichten der Lanzetten in eine Gebrauchsstellung ermöglichen, kann prinzipiell auch ohne Umspritzen mit Kunststoff als Lanzettenrad verwendet werden. Soll-Biegestellen können an den Stegen in einem solchen Fall beispielsweise durch Einprägungen oder eine andere Schwächung des Steges vorgegeben werden.

Bevorzugt sind die Lanzettenspitzen bei einem erfindungsgemäßen Lanzettenrad radial nach außen gerichtet. Auf diese Weise können die Stege strahlenförmig von einem gemeinsamen Zentrum ausgehen, was eine vorteilhaft einfache Fertigung ermöglicht.

Bei einem erfindungsgemäßen Lanzettenrad kann der Kunststoffkörper als Trägerkörper der Lanzetten vorteilhaft ein Lanzettenmagazin bilden, das eine einfache Handhabung ermöglicht und ein Kopplungselement zur Ankopplung an eine Fortschalteinrichtung eines Stechgeräts ausbildet. Bevorzugt wird deshalb beim Umspritzen eines metallenen Lanzettenradrohlings mit Kunststoff ein Trägerkörper erzeugt, der ein Kopplungselement zur Ankopplung an eine Fortschalteinrichtung eines Stechgeräts ausbildet, mit der die Lanzetten des Lanzettenrades nacheinander in ihre Gebrauchsposition bewegt werden können. Im einfachsten Fall kann das Kopplungselement beispielsweise eine Öffnung sein, in die eine Welle einer Fortschalteinrichtung gesteckt werden kann. Ein von dem Kunststoffkörper gebildetes Lanzettenmagazin kann vorteilhaft zudem einen Schutz der Lanzetten bewirken, beispielsweise indem der Trägerkörper einen Ring aufweist, der die Lanzetten umgibt.

Weitere Einzelheiten und Vorteile der Erfindung werden an Ausführungsbeispielen unter Bezugnahme auf die beigefügten Zeichnungen erläutert. Gleiche und einander entsprechende Teile sind dabei mit übereinstimmenden Bezugszahlen gekennzeichnet. Es zeigen:
- Figur 1:: ein Ausführungsbeispiel eines erfindungsgemäßen Lanzettenrads;
- Figur 2:: die Metallteile des in Figur 1 dargestellten Lanzettenrads;
- Figur 3:: ein Ausführungsbeispiel eines Stechsystems mit dem dargestellten Lanzettenrad; und

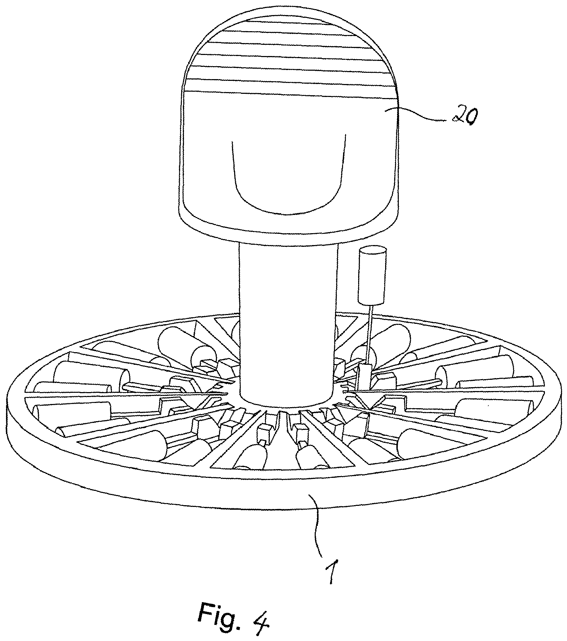

- Figur 4:: das in Figur 1 dargestellte Lanzettenrad mit einem Griff zum Einsetzen in ein Stechgerät.

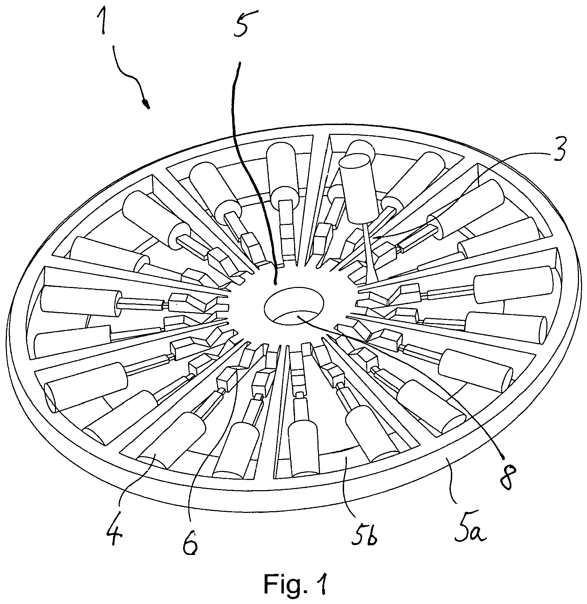

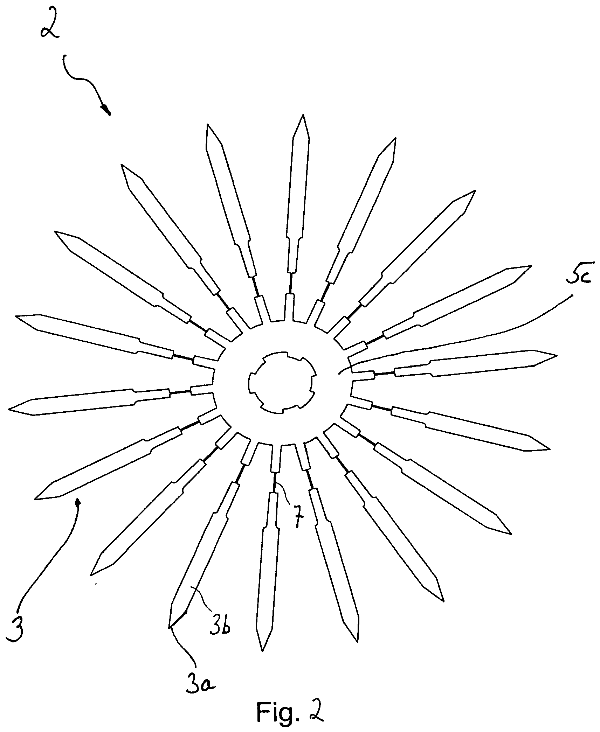

Figur 1 zeigt ein Lanzettenrad 1, das durch Umspritzen des in Figur 2 dargestellten Lanzettenradrohlings 2 mit Kunststoff hergestellt wurde. Das Lanzettenrad 1 bzw. der Lanzettenradrohling 2 haben mehrere ringförmig angeordnete Lanzetten 3, die einen in einer Lanzettenspitze 3a endenden Lanzettenkörper 3b aus Metall aufweisen. Die Lanzettenspitzen 3a sind bei dem in Figur 1 dargestellten Lanzettenrad 1 von einem Sterilschutz 4, beispielsweise aus Schaumstoff, umgeben und deshalb nicht sichtbar.

Das Lanzettenrad 1 hat einen als Rahmen ausgebildeten Träger 5, der die Lanzetten 3 trägt. Die Lanzetten 3 sind über Stege 6 mit dem Träger 5 verbunden, die ein Aufrichten der Lanzetten 3 in eine Gebrauchsstellung ermöglichen. Die Lanzetten 3 führen beim Aufrichten eine Schwenkbewegung durch. In Figur 1 ist beispielhaft eine Lanzette 3 in ihrer aufgerichteten Gebrauchsstellung dargestellt.

Die Stege 6 haben einen Kunststoffkörper, der eine Soll-Biegestelle aufweist, um welche die Stege zum Aufrichten der Lanzetten gebogen werden. Bei dem dargestellten Ausführungsbeispiel ist die Soll-Biegestelle durch eine Kerbe in dem Kunststoffkörper vorgegeben. Die Kunststoffkörper der Stege 6 gehen in den Kunststoffkörper des Trägers 5 über. Die Kunststoffkörper der Stege 6 und der Kunststoffkörper des Trägers 5 werden beim Umspritzen des Lanzettenrohlings 2 als einstückiges Spritzgussteil erzeugt.

In den Kunststoffkörpern der Stege 6 sind von den Lanzettenkörpern 3b ausgehende Metallstege 7 des in Figur 2 dargestellten Lanzettenradrohlings 2 eingebettet. An der Soll-Biegestelle ist der Kunststoffkörper breiter als der Metallsteg 7, vorzugsweise mindestens doppelt so breit, insbesondere mindestens dreimal so breit, bei dem dargestellten Ausführungsbeispiel mehr als fünfmal so breit. Auf diese Weise wird der zum Aufrichten der Lanzetten 3 und somit zum Knicken der Stege 6 erforderliche Kraftaufwand durch den Metallsteg 7 nur unwesentlich erhöht.

Die radial nach außen gerichteten Lanzettenspitzen 3a sind von einem Ring 5a des Trägers 5 umschlossen und auf diese Weise vor unbeabsichtigtem Kontakt bei der Handhabung des Lanzettenrades 1 geschützt. Der Träger 5 weist zudem einen Anschlag 5b in Form einer Ringscheibe auf, der ein Aufrichten der Lanzetten 3 nur in einer Richtung, nämlich weg von der Ringscheibe 5b, zulässt. Auf diese Weise ist die Gefahr eine Beschädigung des Lanzettenrades 2 oder einer Verletzung bei der Handhabung durch ein versehentliches Biegen eines Steges 6 reduziert.

Bei dem dargestellten Ausführungsbeispiel ist ein weiterer Teil des Trägers 5 durch eine Metallscheibe 5c des Lanzettenradrohlings 2 gebildet, von der die Metallstege 7 ausgehen. Die Metallscheibe 5c, bei dem dargestellten Ausführungsbeispiel eine Ringscheibe, ist in den Kunststoffkörper des Trägers 5 durch Umspritzen eingebettet.

Der aus Kunststoff gebildete Trägerkörper 5 des Lanzettenrades 1 bildet bei dem dargestellten Ausführungsbeispiel ein Lanzettenmagazin aus, das die Handhabung der Lanzetten erleichtert und ein Kopplungselement zum Ankoppeln an eine Fortschalteinrichtung eines Stechgeräts ausbildet, mit der die Lanzetten 3 des Lanzettenrades 1 nacheinander in ihre Gebrauchsposition bewegt werden können. Das Kopplungselement kann beispielsweise als ein Loch 8 zum Aufstecken auf eine Welle eines Stechgeräts ausgebildet sein. Das Loch 8 kann als Sackloch oder als durchgehendes Loch in dem Träger 5 angeordnet sein.

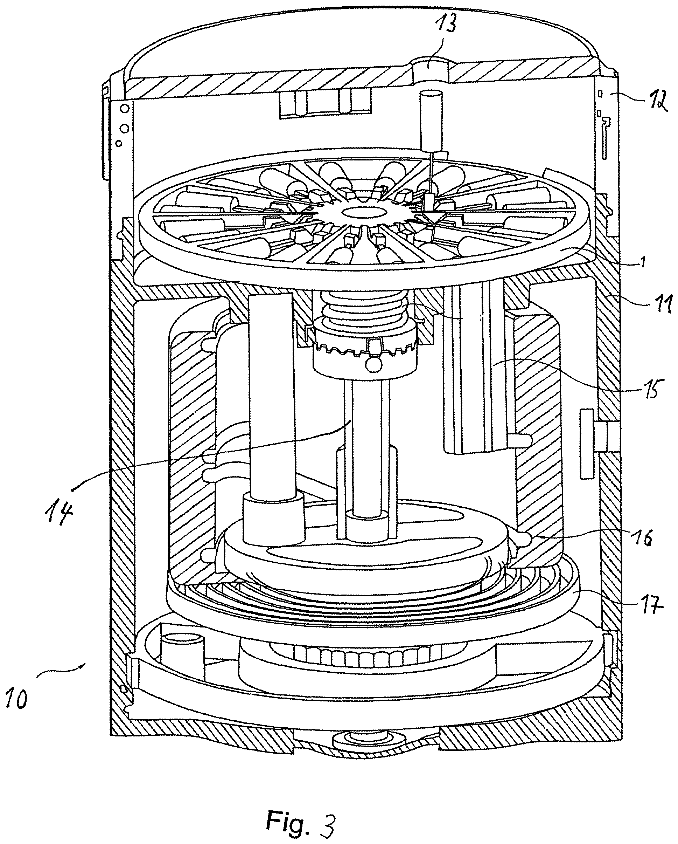

Figur 3 zeigt ein Ausführungsbeispiel eines Stechgeräts 10, das zusammen mit einem Lanzettenrad 1 ein Stechsystem bildet. Das Stechgerät 10 hat ein Gehäuse 11 mit einer zum Austauschen eines Lanzettenrads 1 abnehmbaren Gehäusekappe 12.

In der Gehäusekappe 12 ist eine Gehäuseöffnung 13 angeordnet, an die ein Körperteil zum Erzeugen einer Stichwunde angelegt werden kann.

Das Lanzettenrad 1 ist in dem Stechgerät 10 auf eine Welle 14 gesteckt, mit der die Lanzetten 3 durch Drehen des Lanzettenrads 1 nacheinander zum Gebrauch positioniert werden können. Die Lanzetten 3 werden mit einer Schwenkeinrichtung 15 in die Gebrauchsposition aufgerichtet, die bei dem dargestellten Ausführungsbeispiel als ein in Stichrichtung beweglicher Schieber ausgebildet ist. Wird der Schieber 15 in Stichrichtung verschoben, so trifft er auf eine unter der Öffnung 13 positionierte Lanzette 3, so dass diese in die Gebrauchsposition aufgerichtet wird, in der ihre Lanzettenspitze 3a zu der Gehäuseöffnung 13 deutet. Beim Aufrichten der Lanzette 3 knickt der Steg 6 an der durch die Kerbe definierten Soll-Biegestelle.

Bei einem Stich bewegt der Stechantrieb 16 des dargestellten Stechgeräts 10 das gesamte Lanzettenrad 1 in Stichrichtung. Die Kraft hierfür wird von einer Antriebsfeder 17, beispielsweise eine Spiralfeder, aufgebracht, die über einen Rotor auf eine Kulissensteuerung einwirkt. Die Antriebsfeder wird mittels einer Spannscheibe gespannt, indem diese um 180° gedreht wird. Dabei wird das Lanzettenrad über ein Getriebe automatisch um eine Lanzette 3 weitergetaktet, bei dem dargestellten Ausführungsbeispiel also um 18°, da das Lanzettenrad 1 zwanzig Lanzetten 3 hat.

Der Sterilschutz 4 kann während einer Stichbewegung an der Lanzette 3 bleiben oder vorher entfernt werden. Indem der Sterilschutz 4 als Schaumstoff ausgebildet ist, wird er beim Auftreffen der Lanzette 3 auf ein an die Gehäuseöffnung 13 angelegtes Körperteil zusammen geschoben und behindert deshalb die Lanzettenbewegung allenfalls unwesentlich.

Sobald eine Lanzette 3 nicht mehr von dem Schieber 15 in ihrer aufgerichteten Stellung gehalten wird, schnappt sie wieder in eine ebene Lage zurück.

Um das Einlegen des Lanzettenrads 1 in das Stechgerät 10 zu erleichtern, kann das Lanzettenrad 1 mit einem abnehmbaren Griff 20 versehen werden, wie er in Figur 11 gezeigt ist. Ein verbrauchtes Lanzettenrad 1 kann nach dem Abnehmen der Gehäusekappe 12 mittels eines Auswerfers per Knopfdruck aus dem Stechgerät 10 ausgeworfen werden.

### Bezugszahlen

- 1: Lanzettenrad
- 2: Lanzettenradrohling
- 3: Lanzetten
- 3a: Lanzettenspitze
- 3b: Lanzettenkörper
- 4: Sterilschutz
- 5: Träger
- 5a: Ring
- 5b: Anschlag
- 5c: Metallscheibe
- 6: Steg
- 7: Metallsteg
- 8: Loch
- 10: Stechgerät
- 11: Gehäuse
- 12: Gehäusekappe
- 13: Gehäuseöffnung
- 14: Welle
- 15: Schwenkeinrichtung
- 16: Stechantrieb
- 17: Antriebsfeder
- 20: Griff

## Patentansprüche

1. Lanzettenrad mit
mehreren ringförmig angeordneten Lanzetten (3), die einen in einer Lanzettenspitze (3a) endenden Lanzettenkörper (3b) aus Metall aufweisen, und
einem Träger (5), mit dem die Lanzetten (3) über radial verlaufende Stege (6) verbunden sind, die ein Aufrichten der Lanzetten (3) in eine Gebrauchsstellung ermöglichen,
**dadurch gekennzeichnet, dass** die Lanzetten (3) radial ausgerichtet sind,
dass die Stege (6) einen Kunststoffkörper haben, der eine Soll-Biegestelle vorgibt, um welche die Stege (6) zum Aufrichten der Lanzetten (3) gebogen werden, so dass die Lanzetten (3) beim Aufrichten eine Schwenkbewegung durchführen, und
der Träger (5) einen Ring (5a) aufweist, der die radial nach außen gerichteten Lanzettenspitzen (3a) umschließt.

2. Lanzettenrad nach Anspruch 1, **dadurch gekennzeichnet, dass** die Soll-Biegestelle als eine Kerbe in dem Kunststoffkörper ausgebildet ist.

3. Lanzettenrad nach einem der vorstehenden Ansprüche, **dadurch gekennzeichnet, dass** in dem Kunststoffkörper ein von dem Lanzettenkörper (3b) ausgehender Metallsteg (7) eingebettet ist.

4. Lanzettenrad nach Anspruch 3, **dadurch gekennzeichnet, dass** sich der Metallsteg (7) durch die Soll-Biegestelle hindurch erstreckt.

5. Lanzettenrad nach Anspruch 4, **dadurch gekennzeichnet, dass** der Kunststoffkörper an der Soll-Biegestelle breiter als der Metallsteg (7), vorzugsweise mindestens doppelt so breit, insbesondere mindestens dreimal so breit, ist.

6. Lanzettenrad nach einem der Ansprüche 3 bis 5, **gekennzeichnet durch,** dass der Träger (5) eine Metallscheibe (5c) aufweist, zu der die von den Lanzetten (3) ausgehenden Metallstege (7) führen.

7. Lanzettenrad nach einem der vorstehenden Ansprüche, **dadurch gekennzeichnet, dass** der Träger (5) einen Kunststoffkörper aufweist, der in die Kunststoffkörper der Stege (6) übergeht.

8. Lanzettenrad nach einem der vorstehenden Ansprüche, **dadurch gekennzeichnet, dass** die Stege (6) federnd um die Soll-Biegestelle biegsam sind, so dass eine aufgerichtete Lanzette (3) aus der Gebrauchsposition ohne äußere Krafteinwirkung in eine ebene Lage übergeht.

9. Lanzettenrad nach einem der vorstehenden Ansprüche, **dadurch gekennzeichnet, dass** der Träger (5) auf einer Seite des Lanzettenrades einen Anschlag aufweist, der ein Aufrichten der Lanzetten (3) nur in einer Richtung zulässt.

10. Verfahren zur Herstellung eines Lanzettenrades (1) nach einem der vorstehenden Ansprüche, das mehrere ringförmig angeordnete Lanzetten (3) aufweist, mit folgenden Schritten:
- Herstellen eines Lanzettenradrohlings (2) aus Metall, wobei der Lanzettenradrohling ringförmig angeordnete Lanzetten (3) aufweist, die über Metallstege verbunden sind und einen in einer Spitze endenden Lanzettenkörper (3b) aus Metall aufweisen,
- Umspritzen des Lanzettenradrohlings (2) mit Kunststoff.

11. Verfahren nach Anspruch 10, **dadurch gekennzeichnet, dass** der Lanzettenradrohling (2) aus Blech ist.

12. Verfahren nach Anspruch 10 oder 11, **dadurch gekennzeichnet, dass** durch das Umspritzen des Lanzettenradrohlings (2) mit Kunststoff ein Trägerkörper (5) mit einem die Lanzetten (3) umschließenden Ring (5a) ausgebildet wird.

13. Verfahren nach einem der Ansprüche 10 bis 12, **dadurch gekennzeichnet, dass** durch das Umspritzen des Lanzettenradrohlings (2) mit Kunststoff ein Trägerkörper (5) mit einem Kopplungselement zur Ankopplung an eine Fortschalteinrichtung eines Stechgeräts ausgebildet wird, mit der die Lanzetten (3) des Lanzettenrades (1) nacheinander in die Gebrauchsposition bewegt werden können.

14. Verfahren nach einem der Ansprüche 10 bis 12, **dadurch gekennzeichnet, dass** zumindest die von den Lanzettenkörpern (3b) ausgehenden Metallstege (7) des Lanzettenradrohlings (2) umspritzt werden.

## Claims

1. A lancet wheel comprising
multiple annularly positioned lancets (3), which have a lancet body (3b) that is made of metal and ends in a lancet tip (3a), and
a carrier (5), to which the lancets (3) are connected via radially running webs (6), which allow raising of the lancets (3) into a usage position,
**characterized in that** the lancets (3) are radially aligned and the webs (6) have a plastic body, which predefines an intended bending point, around which the webs (6) are bent to raise the lancets (3) such that the lancet perform a pivoting movement when they are raised, and
the carrier comprises (5) a ring (5a) that encircles the radially outwardly pointing lancet tips (3a).

2. The lancet wheel according to claim 1, **characterized in that** the intended bending point is implemented as a notch in the plastic body.

3. The lancet wheel according to any one of the preceding claims, **characterized in that** a metal web (7) originating from the lancet body (3b) is embedded in the plastic body

4. The lancet wheel according to claim 3, **characterized in that** the metal web (7) extends through the intended bending point.

5. The lancet wheel according to claims 4, **characterized in that** the plastic body is wider at the intended bending point on the metal web (7), preferably at least twice as wide, in particular at least three times as wide.

6. The lancet wheel according to any one of claims 3 to 5, **characterized in that** the carrier (5) has a metal disk (5c), to which the metal webs (7) originating from the lancets (3) lead.

7. The lancet wheel according to any one of the preceding claims, **characterized in that** the carrier (5) has a plastic body, which merges into the plastic bodies of the webs (6).

8. The lancet wheel according to any one of the preceding claims, **characterized in that** the webs (6) are elastically bendable around the intended bending point, so that a raised lancet (3) passes into a flat location from the usage position without external force action.

9. The lancet wheel according to any one of the preceding claims, **characterized in that** the carrier (5) has a stop on one side of the lancet wheel, which only permits raising of the lancets (3) in one direction.

10. A method for producing a lancet wheel (1), which comprises multiple annularly
positioned lancets (3), comprising the following steps:
- producing a lancet wheel blank (2) from metal, the lancet wheel blank comprising annularly positioned lancets (3), which are connected via metal webs and have a lancet body (3b) that is made of metal and ends in a tip,
- injection molding a plastic coating around the lancet wheel blank (2).

11. The method according to claim 10, **characterized in that** the lancet wheel blank (2) is made of sheet metal.

12. The method according to claim 10 or 11, **characterized in that** a carrier body (5) comprising a ring (5a), which encloses the lancets (3), is made by the injection molding of plastic around the lancet wheel blank (2).

13. The method according to any one of claims 10 to 12, **characterized in that** a carrier body (5) comprising a coupling element for coupling on an advance unit of a lancing device, using which the lancets (3) of the lancet wheel (1) may be moved one after another into the usage position, is made by the injection molding of plastic around the lancet wheel blank (2).

14. The method according to any one of claims 10 to 12, **characterized in that** at least the metal webs (7) of the lancet wheel blank (2) originating from the lancet bodies (3b) are coated by injection molding.

## Revendications

1. Roue de lancettes comprenant plusieurs lancettes (3) disposées en cercle qui présentent un corps de lancette (3b) en métal qui se termine en une pointe de lancette (3a), et un support (5) auquel sont reliées les lancettes (3) via des entretoises (6) s'étendant en direction radiale qui permettent un redressement des lancettes (3) jusque dans une position d'utilisation, **caractérisée en ce que** les lancettes (3) sont orientées en direction radiale ; **en ce que** les entretoises (6) possèdent un corps en matière synthétique qui fixe un point de flexion de consigne autour duquel fléchissent les entretoises (6) pour le redressement des lancettes (3) d'une manière telle que les lancettes (3) exécutent un mouvement de pivotement lors du redressement ; et **en ce que** le support (5) présente un anneau (5a) qui entoure les pointes de lancettes (3) orientées vers l'extérieur en direction radiale.

2. Roue de lancettes selon la revendication 1, **caractérisée en ce que** le point de flexion de consigne est réalisé sous la forme d'une encoche dans le corps en matière synthétique.

3. Roue de lancettes selon l'une quelconque des revendications précédentes, **caractérisée en ce qu'**une entretoise métallique (7) qui émane du corps de lancette (3b) est encastrée dans le corps en matière synthétique.

4. Roue de lancettes selon la revendication 3, **caractérisée en ce que** l'entretoise métallique (7) s'étend sur tout le point de flexion de consigne.

5. Roue de lancettes selon la revendication 4, **caractérisée en ce que** le corps en matière synthétique est plus large, au point de flexion de consigne, que l'entretoise métallique (7), de préférence au moins deux fois aussi large, en particulier au moins trois fois aussi large.

6. Roue de lancettes selon l'une quelconque des revendications 3 à 5, **caractérisée en ce que** le support (5) présente un disque métallique (5c) vers lequel se dirigent les entretoises métalliques (7) émanant des lancettes (3).

7. Roue de lancettes selon l'une quelconque des revendications précédentes, **caractérisée en ce que** le support (5) présente un corps en matière synthétique qui se transforme pour devenir les corps en matière synthétique des entretoises (6).

8. Roue de lancettes selon l'une quelconque des revendications précédentes, **caractérisée en ce que** les entretoises (6) manifestent une aptitude à la flexion analogue à celle d'un ressort autour du point de flexion de consigne, d'une manière telle qu'une lancette redressée (3) passe de sa position d'utilisation sans l'intervention d'une force externe dans une position plane.

9. Roue de lancettes selon l'une quelconque des revendications précédentes, **caractérisée en ce que** le support (5) présente, sur un côté de la roue de lancettes, une butée qui autorise un redressement des lancettes (3) dans une seule direction.

10. Procédé pour la fabrication d'une roue de lancettes (1) selon l'une quelconque des revendications précédentes, qui présente plusieurs lancettes (3) disposées en cercle, comprenant les étapes suivantes dans lesquelles :
- on fabrique une ébauche de roue de lancettes (2) en métal, l'ébauche de roue de lancettes présentant des lancettes (3) disposées en cercle qui sont reliées via des entretoises métalliques et qui présentent un corps de lancette (3b) en métal qui se termine en formant une pointe ;
- on enrobe par injection l'ébauche de roue de lancettes (2) avec une matière synthétique.

11. Procédé selon la revendication 10, **caractérisé en ce que** l'ébauche de roue de lancettes (2) est réalisée en tôle.

12. Procédé selon la revendication 10 ou 11, **caractérisé en ce que**, via l'enrobage par injection de l'ébauche de roue de lancettes (2) avec une matière synthétique, on réalise un corps de support (5) comprenant un anneau (5a) entourant les lancettes (3).

13. Procédé selon l'une quelconque des revendications 10 à 12, **caractérisé en ce que**, via l'enrobage par injection de l'ébauche de roue de lancettes (2) avec une matière synthétique, on réalise un corps de support (5) comprenant un élément d'accouplement destiné à l'accouplement à un mécanisme de progression cran par cran d'un appareil de perçage, avec lequel on peut faire passer les lancettes (3) de la roue de lancettes (1) l'une après l'autre dans la position d'utilisation.

14. Procédé selon l'une quelconque des revendications 10 à 12, **caractérisé en ce qu'**on enrobe par injection au moins les entretoises métalliques (7) de l'ébauche de roue de lancettes (2), émanant des corps de lancettes (3b).
